# EUROPÄISCHE PATENTSCHRIFT

(11) **EP 0 412 359 B1**
(45) Veröffentlichungstag und Bekanntmachung des Hinweises auf die Patenterteilung: **08.06.1994**
(21) Anmeldenummer: 90114304.0
(22) Anmeldetag: 26.07.1990
(51) Int. Cl.: C07D 303/34

(54) **Verfahren zur Herstellung von Epoxysulfonaten**
Method for the preparation of epoxysulphonates
Procédé de préparation d'époxysulfonates

(30) Priorität: 08.08.1989 DE 3926117
(43) Veröffentlichungstag der Anmeldung: 13.02.1991
(73) Patentinhaber: BAYER AG, 51368 Leverkusen (DE)
(72) Erfinder: Schröder, Georg, Dr., D-5093 Burscheid (DE); Arlt, Dieter, Prof. Dr.,, D-5000 Köln 80 (DE); Jautelat Manfred, Dr., D-5093 Burscheid (DE)

(56) Entgegenhaltungen:
- EP-A- 0 351 332
- CHEMICAL ABSTRACTS, Band 79, Nr. 3, 23. Juli 1973, Columbus, Ohio, USAWOJTOWICZ, J.A. et al. "3-Substituted oxetanes" Seite 434, Spalte 2, Zu-sammenfassung-Nr. 18 510g
- CHEMICAL ABSTRACTS, Band 71, Nr. 25, 13. Oktober 1969, Columbus, Ohio, USAHAVBRANDT, OLAVI et al. "Synthesis of 2-methanesul- fonyloxy-1-propanol andsome related esters" Seite 255, Spalte 1, Zu- sammenfassung-Nr. 70 032b
- CHEMICAL ABSTRACTS, Band 69, Nr. 13, 23. September 1968, Columbus, Ohio, USACHAUTEMPS, PIERRE et al. "Preparation of ethylenic alcohol and epoxy alcoholsulfonates" Seite 4841, Spalte 1, Zu- sammenfassung-Nr. 51 899g
- CHEMICAL ABSTRACTS, Band 65, Nr. 1, 4. Juli 1966, Columbus, Ohio, USA NOBUONAKABAYASHI et al. "Some reactions of the glycidyl esters of sulfonic acids"Spalte 671, Zusammenfassung- Nr. 671d
- CHEMICAL ABSTRACTS, Band 57, Nr. 6, 17. September 1962, Columbus, Ohio, USAKURT MEYER "Twenty-five years of the Agfacolor process" Spalte 6785,Zusammenfassung -Nr. 6 785a
- CHEMICAL ABSTRACTS, Band 100, Nr. 13, 26. MUrz 1984, Columbus, Ohio, USANKUNYA, M. H. H. et al. "Asymmetric synthesis of alpha,beta-epoxysulfonateesters (oxiranesulfonate esters)" Seite 625, Spalte 1, Zu- sammenfassung-Nr.103 075r

## Beschreibung

Es ist bekannt, daß Epoxysulfonate aus den entsprechenden Epoxyalkoholen durch Veresterung mit Sulfonsäurechloriden zugänglich sind [D.E. Mc Clure, J.J. Baldwin, A.W. Raab, K. Mensler, B.H. Arison, J. Org. Chem., 43, 4876 (1978)]. Die Epoxyalkohole sind aber im technischen Maßstab nicht in jedem Falle preiswert erhältlich, außerdem werden stöchiometrische Mengen organischer Hilfsbasen benötigt. Die Herstellung aus Alkenyloxysulfonaten ist nicht in jedem Falle ausbeutemäßig befriedigend, außerdem werden für die Epoxidierung Persäuren eingesetzt [R. Chautemps, J.L. Pierre, P. Arnaud, C.R. Acad. Sci. Paris, Ser. C., 266, 622 (1968)], die teuer und problematisch bei der Handhabung sind.

Allgemein sind alternative Verfahren zur Herstellung von Sulfonaten bekannt, so z.B. durch Reaktion von Halogeniden mit Silbersulfonaten [P.W. Feit, O.T. Nielson, J. Med. Chem., 9, 416 (1966)]. Die Herstellung über Silbersalze ist aus Kostengründen nicht für die industrielle Herstellung von Epoxysulfonaten geeignet, ebenso die Herstellung über Kupfer-(I)-Sulfonsäuresalze [JP 59 73,587; CA 101, 130519]. Üblicherweise werden aus Sulfonaten jedoch Jodide und Chloride dargestellt [D. Landini, F. Montanari, F. M. Pirisi, J.C.S., Chem. Commun., 1974, 879]. Die katalytische Umwandlung von Alkylchloriden in Jodide [R.C. Hahn, J. Org. Chem., 53, 1331, (1988)] oder in Bromide [P. Tundo, F. Trotta, G. Moraglio, J. Chem. Soc., Perkin Trans. II, 1988, 1709] ist ebenfalls bekannt. Als Alternative zur Herstellung von Epoxysulfonaten wird der katalysierte nukleophile Austausch von Halogeniden in Epoxyhalogeniden vorgeschlagen. Diese Reaktion wurde bislang lediglich bei einfachen Alkylhalogeniden eingesetzt [R.C. Hahn, J. Org. Chem., 53, 5783 (1988)] um diese in Alkylsulfonsäureester zu überführen. Bei der Einwirkung nukleophiler Reagentien auf Epoxide muß stets mit einer Epoxidöffnung [A. S. Rao, S.K. Paknikar, J.G. Kirtane, Tetrahedron, 39, 2323 (1983); M. Caron, P.R. Carlier, K.B. Sharpless, J. Org. Chem., 53, 5185 (1988); D. Klamann, P. Weyerstahl, F. Nerdel, Liebigs Ann. Chem., 710, 59 (1967)] gerechnet werden, so daß die Herstellung von Epoxysulfonaten durch nukleophilen Halogenaustausch, wie hier vorgeschlagen, in Epoxyhalogeniden schwierig sein sollte.

Gegenstand der vorliegenden Erfindung ist ein Verfahren zur Herstellung von Epoxysulfonaten, das dadurch gekennzeichnet ist, daß man ein Epoxyhalogenid der allgemeinen Formel 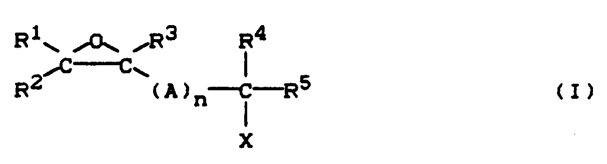
worin R¹ - R⁵ unabhängig voneinander für Wasserstoff oder eine Alkylgruppe mit 1 - 18 C-Atomen, eine Cycloalkylgruppe mit 5 - 14 C-Atomen, einen Arylrest mit 6 - 20 C-Atomen oder eine Aralkylgruppe mit 7 - 16 C-Atomen, A für eine Gruppe 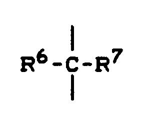
steht, worin R⁶ und R⁷ unabhängig voneinander für Wasserstoff oder eine Alkylgruppe mit 1 - 18 C-Atomen, eine Cycloalkylgruppe mit 5 - 14 C-Atomen, einen Arylrest mit 6 - 20 C-Atomen oder eine Aralkylgruppe mit 7 - 16 C-Atomen, n für eine Zahl von 0 - 8, X für Chlor, Brom oder Jod steht,
mit einem Sulfonsäureester der allgemeinen Formel 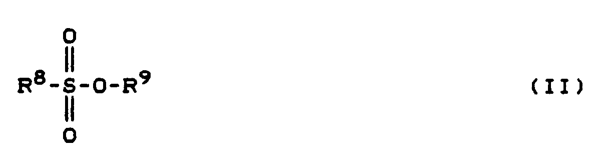
worin R⁸ und R⁹ unabhängig voneinander für eine Alkylgruppe mit 1 - 18 C-Atomen, eine Cycloalkylgruppe mit 5 - 14 C-Atomen, einen Arylrest mit 6 - 20 C-Atomen oder eine Aralkylgrupps mit 7 - 16 C-Atomen steht, in Gegenwart eines Katalysators der allgemeinen Formel 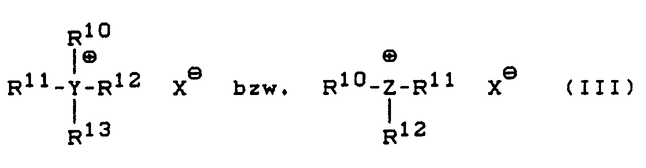
worin R¹⁰ bis R¹³ unabhängig voneinander für eine Alkylgruppe mit 1 - 18 C-Atomen, eine Cycloalkylgruppe mit 5 - 14 C-Atomen, einen Arylrest mit 6 - 20 C-Atomen oder eine Ar-alkylgruppe mit 7 - 16 C-Atomen direkt oder über ein Brückenglied oder R¹⁰ für den Rest eines Polymeren steht, welches
an ein C-Atom der Polymerkette mindestens eine Gruppe 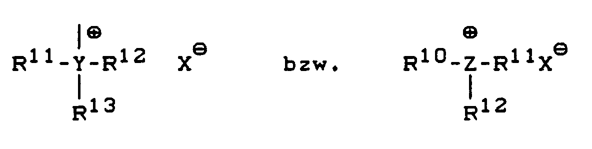
gebunden enthält,
- Y: für Stickstoff, Phosphor oder Arsen, Z für S oder S=O
- X: für Chlor, Brom Jod oder R⁸SO₃^{⊖} steht
zu einem Epoxysulfonat der allgemeinen Formel 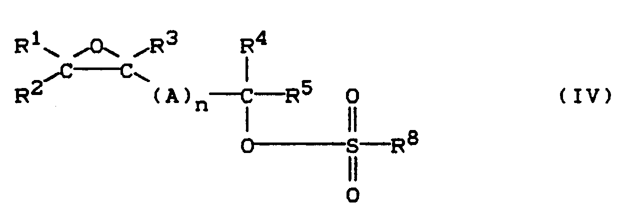
umsetzt.

Vorzugsweise setzt man 1 Mol (I) mit 0,1 - 10 Mol (II) in Gegenwart von 0,001 - 0,5 Mol (III) bei etwa 50 - 200°C um.

Die Umsetzung erfolgt gegebenenfalls in Gegenwart inerter Verdünnungsmittel, z.B. N,N-Dimethylformamid, N,N-Dimethylacetamid, Dimethylsulfoxid, N-Methylpyrrolidin, Dimethoxyethan, Diethylen-glykoldimethylether usw.. Die Reaktionszeiten liegen im allgemeinen bei etwa 80 Stunden in Abhängigkeit von Temperatur und Katalysatorkonzentration. Die Abtrennung von (IV) erfolgt vorzugsweise durch Destillation, kann jedoch auch chromatographisch oder durch Kristallisation erfolgen. Es kann auch zweckmäßig sein im Verlaufe der Reaktion die Menge an (III) nachträglich schrittweise zu erhöhen.

Bevorzugte Verbindungen (I) sind solche in denen R¹-R⁵ unabhängig voneinander für Wasserstoff oder eine Alkylgruppe mit 1 - 4 C-Atomen, eine Cycloalkylgruppe mit 5 - 6 C-Atomen, einen Arylrest mit 6 - 10 C-Atomen oder eine Aralkylgruppe mit 7 - 10 C-Atomen, A für eine Gruppe R⁶-C-R⁷, worin R⁶ und R⁷ unabhängig voneinander für Wasserstoff oder eine Alkylgruppe mit 1 - 4 C-Atomen, eine Cycloalkylgruppe mit 5 - 6 C-Atomen, einen Arylrest mit 6 - 10 C-Atomen oder eine Aralkylgruppe mit 7 - 10 C-Atomen, n für eine Zahl von 0 bis 2 und X für Chlor, Brom oder Jod steht.

Als Ausgangsverbindungen seien im einzelnen genannt: 1-Chlor-2,3-epoxybutan, 1-Brom-2,3-epoxybutan, 2-Chlor-3,4-epoxy-butan, 2-Brom-3,4-epoxybutan, 1-Chlor-3,4-epoxybutan, 1-Brom-3,4-epoxybutan, 1-Chlor-5,6-epoxyhexan, 1-Brom-5,6-epoxyhexan, 1-Chlor-2-methyl-2,3-epoxypropan und 1-Brom-2-methyl-2,3-epoxypropan. Als besonders bevorzugt seien 1-Chlor-2,3-epoxypropan und 1-Brom-2,3-epoxypropan genannt.

Als Verbindungen der Formel (II) werden bevorzugt solche eingesetzt, worin R⁸ und R⁹ unabhängig voneinander für eine Alkylgruppe mit 1 - 6 C-Atomen, eine Cycloalkylgruppe mit 5 - 6 C-Atomen, einen Arylrest mit 6 - 10 C-Atomen oder eine Aralkylgruppe mit 7 - 10 C-Atomen stehen.

Im einzelnen seien genannt: Methansulfonsäurepropylester, Ethansulfonsäuremethylester, Ethansulfonsäureethylester, Ethansulfonsäurepropylester, Propansulfonsäuremethylester, Propansulfonsäureethylester, Propansulfonsäurepropylester, 4-Toluolsulfonsäuremethylester, 4-Toluolsulfonsäureethylester, 4-Toluolsulfonsäurepropylester, Benzolsulfonsäuremethylester, Benzolsulfonsäureethylester, Benzolsulfonsäurepropylester. Als besonders bevorzugt seien hier Methansulfonsäuremethylester und Methansulfonsäureethylester genannt.

Als Verbindungen der Formel (III) werden bevorzugt solche eingesetzt, worin R¹⁰ bis R¹³ unabhängig voneinander für eins Alkylgruppe mit 1 - 12 C-Atomen, eine Cycloalkylgruppe mit 5 - 10 C-Atomen, einen Arylrest mit 6 - 10 C-Atomen oder eine Aralkylgruppe mit 7 - 10 C-Atomen, oder aber R¹⁰ für ein polymeres Gerüst, Y für Stickstoff, Phosphor oder Arsen, Z für S oder S=O, X für Chlor, Brom, Jod oder B, worin B für die Formel R¹⁴--SO₃ und R¹⁴ für eine Alkylgruppe mit 1 - 12 C-Atomen, eine Cycloalkylgruppe mit 5 - 10 C-Atomen, einen Arylrest mit 6 - 10 C-Atomen oder eine Aralkylgruppe mit 7 - 10 C-Atomen stehen.

Als Ausgangsverbindungen seien im einzelnen genannt: Tetramethylammoniumchlorid, Tetramethylammoniumbromid, Tetramethylammoniumjodid, Tetramethylammoniummethansulfonat, Tetraethylammoniumchlorid, Tetraethylammoniumbromid, Tetraethylammoniumjodid, Tetraethylammoniummethansulfonat, Tetrapropylammoniumchlorid, Tetrapropylammoniumbromid, Tetrapropylammoniumjodid, Tetrapropylammoniummethansulfonat, Tetrabutylammoniumchlorid, Tetrahexylammoniumchlorid, Tetrahexylammoniumbromid, Tetrahexylammoniummethansulfonat, Tetrabutylphosphoniumchlorid, Tetrabutylphosphoniumjodid, Tetrabutylphosphoniummethansulfonat. Als besonders bevorzugt seien hier Tetrabutylammoniumbromid, Tetrabutylammoniumjodid, Tetrabutylammoniummethansulfonat, Tetrahexylammoniumjodid und Tetrabutylphosphoniumbromid aufgeführt.

Geeignete hochmolekulare Verbindungen (III) sind beispielsweise durch Umsetzung von gegebenenfalls vernetzten (z.B. mit Divinylbenzol) chlormethylierten Styrol-Homopolymerisaten bzw. Styrol-Copolymerisaten mit Verbindungen der Formel 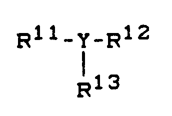
bzw. R¹¹-Z-R¹² erhältlichen Produkte.

Hochmolekulare Verbindungen dieses Typs sind bekannt, vgl. z.B. P. Tundo, u.a.; Synthesis, 1978, 315; J. Chem. Soc., Chem. Communications, 1976, 394.

Es muß als ausgesprochen überraschend bezeichnet werden, daß der nukleophile Austausch von Halogenen in Epoxyhalogeniden durch Sulfonate möglich ist, denn in Gegenwart der reaktiven Epoxygruppe ist mit einer nukleophilen Epoxidöffnung zu rechnen [A.S. Rao, S.K. Paknikar, J.G. Kirtane, Tetrahedron, 39, 2323 (1983); M. Caron, P.R. Carlier, K.B. Sharpless, J. Org. Chem., 53, 5185 (1988), D. Klamann u.a., Liebigs Ann. Chem., 710, 59, 1967]. Durch die dargelegten Maßnahmen gelingt es jedoch die nukleophile Öffnung des Epoxidringes zu vermeiden und so die Epoxysulfonate (IV) darzustellen.

Das erfindungsgemäße Verfahren ermöglicht die Herstellung von Epoxysulfonaten (IV) aus den technisch gut zugänglichen Epoxyhalogeniden (I).

Die Epoxysulfonate sind bekannt.

Durch Umsetzung mit Polyamidaminen oder Polyaminen lassen sich in Analogie zur Umsetzung mit Epihalogenhydrinen Papierausrüstungsmittel erhalten.

Geeignete Ausgangskomponenten und Verfahrensbedingungen sind beispielsweise in folgenden Literatursellen beschrieben: DE-A 22 29 219, 17 71 043, 22 57 271, 29 38 588, 29 49 870, 17 20 905, 19 064 50, EP-A 0 131 200, 0 126 176, US-PS 29 26 154 und 33 32 901.

### Beispiele

### Beispiel 1

Herstellung von 2,3-Epoxypropyl-methansulfonat aus 1-Chlor-2,3-epoxypropan und Methansulfonsäureethylester mit Tetrabutylammoniumbromid als Katalysator
Eine Lösung von 36.98 g (0.4 mol) 1-Chlor-2,3-epoxypropan und 25.0 g (0.2 mol) Methansulfonsäureethylester wird mit 6.45 g (0.02 mol) Tetrabutylammoniumbromid für 60 Stunden auf 120°C erhitzt. Danach wird i. Vak bei 20 mbar 1-Chlor-2,3-epoxypropan und Methansulfonsäureethylester abdestilliert und danach das 2,3-Epoxypropylmethansulfonat destilliert. Man gewinnt so 18.63 g (0.20 mol) 1-Chlor-2,3-epoxypropan und 6.20 g (0.05 mol) Methansulfonsäureethylester zurück.

| | |
|---|---|
| Ausbeute: | 15.75 g (52 % d. Th.) |
| Kp.: | 90-93°C (0.05 mbar) |
| Reinheit: | 86 % (GC) |

### Beispiel 2

Herstellung von 2,3-Epoxypropyl-methansulfonat aus 1-Chlor-2,3-epoxypropan und Methansulfonsäuremethylester mit Tetrabutylammoniumbromid als Katalysator
Eine Lösung von 4.60 g (0.05 mol) 1-Chlor-2,3-epoxypropan und 5.50 (0.05 mol) Methansulfonsäuremethylester wird mit 1.61 g (0.005 mol) Tetrabutylammoniumbromid für 64 Stunden auf 120°C erhitzt. Danach wird i. Vak bei 20 mbar 1-Chlor-2,3-epoxypropan abdestilliert und danach das 2,3-Epoxypropyl-methansulfonat destilliert.

| | |
|---|---|
| Ausbeute: | 3.40 g (45 % d. Th.) |
| Reinheit: | 86 % (GC) |

### Beispiel 3

Herstellung von 2,3-Epoxypropyl-methansulfonat aus 1-Chlor-2,3-epoxypropan und Methansulfonsäuremethylester mit Tetrabutylammoniumbromid als Katalysator
Eine Lösung von 18.5 g (0.2 mol) 1-Chlor-2,3-epoxypropan und 11.0 g (0.1 mol) Methansulfonsäuremethylester wird mit 4.85 g (0.015 mol) Tetrabutylammoniumbromid für 40 Stunden auf 120°C erhitzt. Danach wird i. Vak bei 20 mbar 1-Chlor-2,3-epoxypropan abdestilliert und danach das 2,3-Epoxypropyl-methansulfonat destilliert.

| | |
|---|---|
| Ausbeute: | 10.60 g (70 % d. Th.) |
| Reinheit: | 94 % (GC) |

### Beispiel 4

Herstellung von 2,3-Epoxypropyl-methansulfonat aus 1-Chlor-2,3-epoxypropan und Methansulfonsäuremethylester mit Tetrabutylammoniumbromid als Katalysator
Eine Lösung von 18.5 g (0.2 mol) 1-Chlor-2,3-epoxypropan und 11.0 g (0.1 mol) Methansulfonsäuremethylester wird mit 8.06 g (0.025 mol) Tetrabutylammoniumbromid für 16 Stunden auf 120°C erhitzt. Danach wird i. Vak bei 20 mbar 1-Chlor-2,3-epoxypropan abdestilliert und danach das 2,3-Epoxypropyl-methansulfonat destilliert.

| | |
|---|---|
| Ausbeute: | 9.85 g (65 % d. Th.) |
| Reinheit: | 96 % (GC) |

### Beispiel 5

Herstellung von 2,3-Epoxypropyl-methansulfonat aus 1-Chlor-2,3-epoxypropan und Methansulfonsäuremethylester mit Tetrabutylammoniumjodid als Katalysator
Eine Lösung von 9.25 g (0.1 mol) 1-Chlor-2,3-epoxypropan und 11.0 g (0.1 mol) Methansulfonsäuremethylester wird mit 3.70 g (0.01 mol) Tetrabutylammoniumjodid für 64 Stunden auf 120°C erhitzt. Danach wird i. Vak bei 20 mbar 1-Chlor-2,3-epoxypropan abdestilliert und danach das 2,3-Epoxypropyl-methansulfonat destilliert.

| | |
|---|---|
| Ausbeute: | 7.60 g (50 % d. Th.) |
| Reinheit: | 75 % (GC) |

### Beispiel 6

Herstellung von 2,3-Epoxypropyl-methansulfonat aus 1-Chlor-2,3-epoxypropan und Methansulfonsäuremethylester mit Tetrabutylammoniummethansulfonat als Katalysator
Eine Lösung von 9.25 g (0.1 mol) 1-Chlor-2,3-epoxypropan und 11.0 g (0.1 mol) Methansulfonsäuremethylester wird mit 3.38 g (0.01 mol) Tetrabutylammoniummethansulfonat für 64 Stunden auf 120°C erhitzt. Danach wird i. Vak bei 20 mbar 1-Chlor-2,3-epoxypropan abdestilliert und danach das 2,3-Epoxypropylmethansulfonat destilliert.

| | |
|---|---|
| Ausbeute: | 9.25 g (61 % d. Th.) |
| Reinheit: | 77 % (GC) |

### Beispiel 7

Herstellung von 2,3-Epoxypropyl-methansulfonat aus 1-Chlor-2,3-epoxypropan und Methansulfonsäuremethylester mit Tetrabutylphosphoniumbromid als Katalysator
Eine Lösung von 9,25 g (0.1 mol) 1-Chlor-2,3-epoxypropan und 11.0 g (0.1 mol) Methansulfonsäuremethylester wird mit 3.40 g (0.01 mol) Tetrabutylammoniumjodid für 64 Stunden auf 120°C erhitzt. Danach wird i. Vak bei 20 mbar 1-Chlor-2,3-epoxypropan abdestilliert und danach das 2,3-Epoxypropyl-methansulfonat destilliert.

| | |
|---|---|
| Ausbeute: | 6.30 g (61 % d. Th.) |
| Reinheit: | 75 % (GC) |

### Beispiel 8

Herstellung von 2,3-Epoxypropyl-methansulfonat aus 1-Chlor-2,3-epoxypropan und Methansulfonsäuremethylester mit DOWEX 1X8 (= basischer Anionenaustauscher: mit Methylbromid quaterniertes, mit ca. 8 % Divinylbenzol vernetztes Dimethylaminomethylstyrol) als Katalysator.

Eine Lösung von 9.25 g (0.1 mol) 1-Chlor-2,3-epoxypropan und 5.50 g (0.05 mol) Methansulfonsäuremethylester wird mit 3.00 g des Anionenaustauschers DOWEX 1X8 (Br^{⊖}-Form) für 64 Stunden auf 120°C erhitzt. Danach wird i. Vak bei 20 mbar 1-Chlor-2,3-epoxypropan abdestilliert und danach das 2,3-Epoxypropyl-methansulfonat destilliert.

| | |
|---|---|
| Ausbeute: | 3.50 g (46 % d. Th.) |
| Reinheit: | 75 % (GC) |

### Beispiel 9

Herstellung von 2,3-Epoxypropyl-methansulfonat aus 1-Chlor-2,3-expoxypropan und Methansulfonsäuremethylester mit Anionanaustauscher (mit Tributylphosphin quaterniertes Polybromohexylstyrol; vgl. P. Tundo, Synthesis 315, 1978) als Katalysator.

Eine Lösung von 6.13 g (0.066 mol) 1-Chlor-2,3-epoxypropan und 3.67 (0.033 mol) Methansulfonsäuremethylester wird mit 2.00 g des Anionenaustauschers (Br^{⊖}-Form; vgl. P. Tundo, Synthesis, 315, 1978) für 64 Stunden auf 120°C erhitzt. Danach wird i. Vak bei 20 mbar 1-Chlor-2,3-epoxypropan abdestilliert und danach das 2,3-Epoxypropyl-methensulfonat destilliert.

| | |
|---|---|
| Ausbeute: | 3.60 g (71 % d. Th.) |
| Reinheit: | 71 % (GC) |

### Beispiel 10

Herstellung von 2,3-Epoxypropyl-methansulfonat aus 1-Chlor-2,3-epoxypropan und Methansulfonsäuremethylester mit einen basischen Anionenaustauscher (mit Methylbromid quarterniertes mit ca. 6 % Divinylbenzol vernetztes Polydimethylaminostyrol; vgl. DE-A 1 054 715) als Katalysator.

Eine Lösung von 9.25 g (0.1 mol) 1-Chlor-2,3-epoxypropan und 5.50 g (0.05 mol) Methansulfonsäuremethylester wird mit 3.00 g des Anionenaustauschers (Br^{⊖}-Form (vgl. DE-A-1 054 715) für 40 Stunden auf 120°C erhitzt. Danach wird i. Vak bei 20 mbar 1-Chlor-2,3-epoxypropan abdestilliert und danach das 2,3-Epoxypropyl-methansulfonat destilliert.

| | |
|---|---|
| Ausbeute: | 4.60 g (61 % d. Th.) |

## Patentansprüche

1. Verfahren zur Herstellung von Epoxysulfonaten der Formel 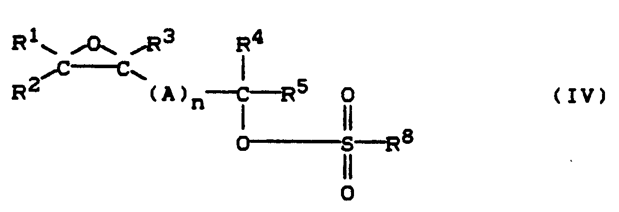 dadurch gekennzeichnet, daß man ein Epoxyhalogenid der Formel 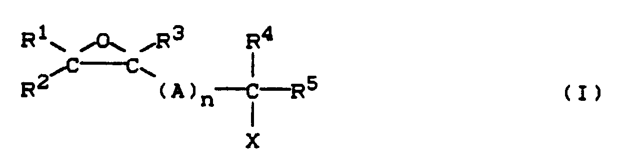 worin R¹ - R⁵ unabhängig voneinander für Wasserstoff oder eine Alkylgruppe mit 1 - 18 C-Atomen, eine Cycloalkylgruppe mit 5 - 14 C-Atomen, einen Arylrest mit 6 -20 C-Atomen oder eine Aralkylgruppe mit 7 - 16 C-Atomen,
A für eine Gruppe 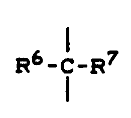 steht, worin R⁶ und R⁷ unabhängig voneinander für Wasserstoff oder eine Alkylgruppe mit 1 - 18 C-Atomen, eine Cycloalkylgruppe mit 5 - 14 C-Atomen, einen Arylrest mit 6 - 20 C-Atomen oder eine Aralkylgruppe mit 7 - 16 C-Atomen, n für eine Zahl von 0 - 8, X für Chlor, Brom oder Jod steht,
mit einem Sulfonsäureester der allgemeinen Formel 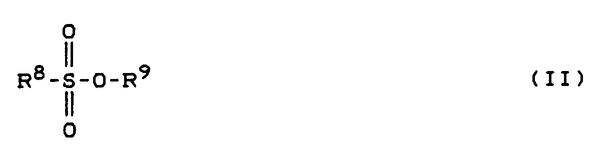 worin R⁸ und R⁹ unabhängig voneinander für eine Alkylgruppe mit 1 - 18 C-Atomen, eine Cycloalkylgruppe mit 5 - 14 C-Atomen, einen Arylrest mit 6 - 20 C-Atomen oder eine Aralkylgruppe mit 7 - 16 C-Atomen steht, in Gegenwart eines Katalysators der allgemeinen Formel 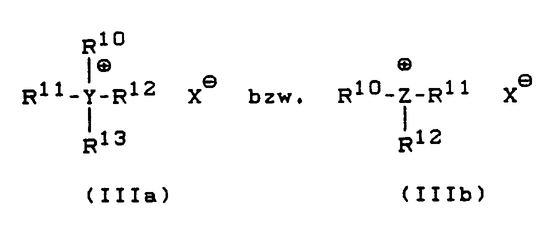 worin R¹⁰ bis R¹³ unabhängig voneinander für eine Alkylgruppe mit 1 - 18 C-Atomen, eine Cycloalkylgruppe mit 5 - 14 C-Atomen, einen Arylrest mit 6 - 20 C-Atomen oder eine Ar-alkylgruppe mit 7 - 16 C-Atomen direkt oder über ein Brückenglied oder R₁₀ für den Rest eines Polymeren steht, welches
an ein C-Atom der Polymerkette mindestens eine Gruppe 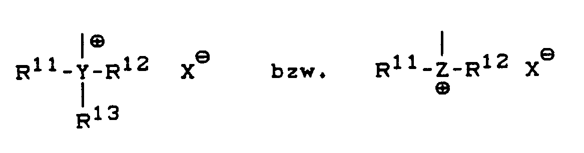 gebunden enthält,
Y für Stickstoff, Phosphor oder Arsen, Z für S oder S=O
X für Chlor, Brom Jod oder R⁸SO₃^{⊖} steht, umsetzt.

2. Verfahren nach Anspruch 1, dadurch gekennzeichnet, daß man bei 50-200°C mit 0,001-0,5 Mol Katalysator umsetzt.

## Claims

1. Process for the preparation of epoxysulphonates of the formula 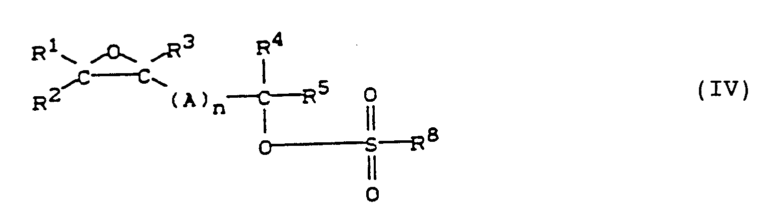 characterized in that an epoxyhalide of the formula 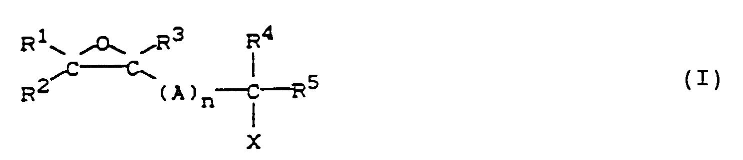 wherein R¹ - R⁵ independently of one another represent hydrogen or an alkyl group having 1 - 18 C atoms, a cycloalkyl group having 5 - 14 C atoms, an aryl radical having 6 - 20 C atoms or an aralkyl group having 7 - 16 C atoms, A represents a group 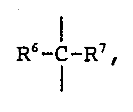 wherein R⁶ and R⁷ independently of one another represent hydrogen or an alkyl group having 1 - 18 C atoms, a cycloalkyl group having 5 - 14 C atoms, an aryl radical having 6 - 20 C atoms or an aralkyl group having 7 - 16 C atoms, n represents a number from 0 to 8 and X represents chlorine, bromine or iodine,
is reacted with a sulphonic acid ester of the general formula 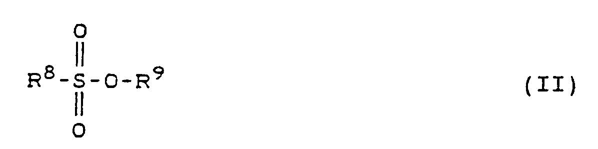 wherein R⁸ and R⁹ independently of one another represent an alkyl group having 1 - 18 C atoms, a cycloalkyl group having 5 - 14 C atoms, an aryl radical having 6 - 20 C atoms or an aralkyl group having 7 - 16 C atoms, in the presence of a catalyst of the general formula 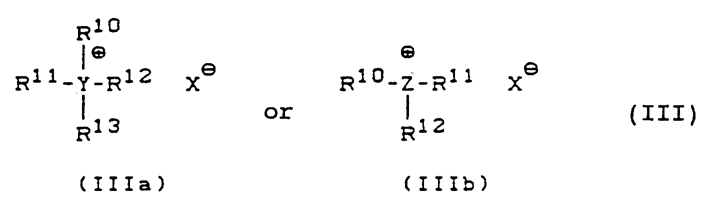 wherein R¹⁰ to R¹³ independently of one another represent an alkyl group having 1 - 18 C atoms, a cycloalkyl group having 5 - 14 C atoms, an aryl radical having 6 - 20 C atoms or an aralkyl group having 7 - 16 C atoms, attached directly or via a bridge member, or R¹⁰ represents the radical of a polymer which
contains at least one group 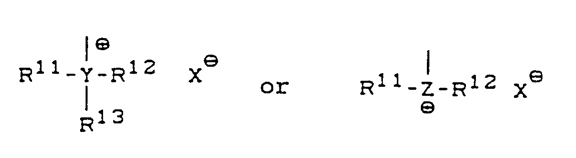 bonded to a C atom of the polymer chain,
Y represents nitrogen, phosphorus or arsenic, Z represents S or S=O and X represents chlorine, bromine, iodine or R⁸SO₃^{⊖}.

2. Process according to Claim 1, characterized in that the reaction is carried out at 50-200°C using 0.001-0.5 mol of catalyst.

## Revendications

1. Procédé de préparation d'époxysulfonates de formule 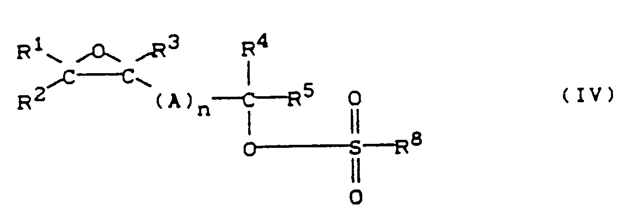 caractérisé en ce que l'on transforme un époxyhalogénure de formule générale 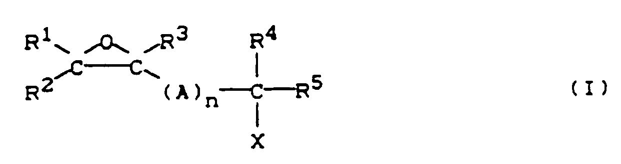 dans laquelle les R¹ - R⁵ représentent indépendamment les uns des autres l'hydrogène ou un groupe alkyle de 1 à 18 atomes de carbone, un groupe cycloalkyle de 5 à 14 atomes de carbone, un reste aryle de 6 à 20 atomes de carbone ou un groupe aralkyle de 7 à 16 atomes de carbone,
A représente un groupe 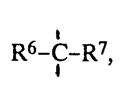 dans lequel R⁶ et R⁷ sont indépendamment l'un de l'autre l'hydrogène ou un groupe alkyle de 1 à 18 atomes de carbone, un groupe cycloalkyle de 5 à 14 atomes de carbone, un reste aryle de 6 à 20 atomes de carbone ou un groupe aralkyle de 7 à 16 atomes de carbone, n est un nombre de 0 à 8, X représente le chlore, le brome ou l'iode,
avec un ester d'acide sulfonique de formule générale 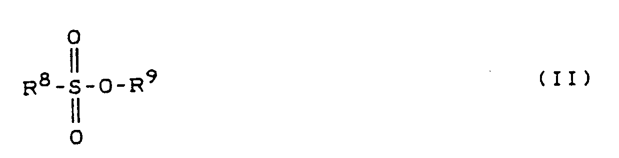 dans laquelle R⁸ et R⁹ représentent indépendamment l'un de l'autre un groupe alkyle de 1 à 18 atomes de carbone, un groupe cycloalkyle de 5 à 14 atomes de carbone, un reste aryle de 6 à 20 atomes de carbone ou un groupe aralkyle de 7 à 16 atomes de carbone, en présence d'un catalyseur de formule générale 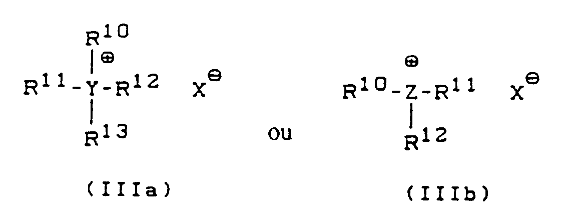 où R¹⁰ à R¹³ représentent indépendamment les uns des autres un groupe alkyle de 1 à 18 atomes de carbone, un groupe cycloalkyle de 5 à 14 atomes de carbone, un reste aryle de 6 à 20 atomes de carbone ou un groupe aralkyle de 7 à 16 atomes de carbone, reliés directement ou par l'intermédiaire d'un pont, ou R¹⁰ représente le reste d'un polymère qui contient au moins un groupe 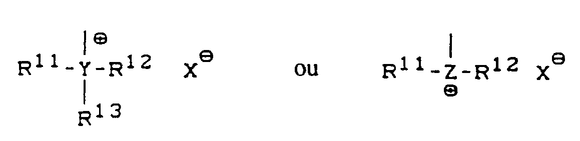 lié à un atome de carbone de la chaîne polymère,
Y représente l'azote, le phosphore ou l'arsenic, Z est S ou S=O,
X représente le chlore, le brome, l'iode ou R⁸SO₃^{⊖}.

2. Procédé selon la revendication 1, caractérisé en ce que l'on effectue la réaction à 50 - 200°C avec 0,001 - 0,5 mole de catalyseur.
